# EUROPEAN PATENT APPLICATION

(11) **EP 1 983 346 A1**
(43) Date of publication of application: **22.10.2008**
(21) Application number: 07708225.3
(22) Date of filing: 08.02.2007
(51) Int. Cl.: G01N 33/68, C07K 1/06

(54) **METHOD FOR CLEAVAGE OF PEPTIDE BOND AT C-TERMINUS OF PEPTIDE, AND METHOD FOR DETERMINATION OF C-TERMINAL AMINO ACID SEQUENCE OF PEPTIDE**

(30) Priority: 08.02.2006 JP 2006031097
(71) Applicant: NEC Corporation, Minato-ku Tokyo (JP)
(72) Inventor: MIYAZAKI, Kenji, Tokyo 108-0014 (JP); KAMIJO, Ken'ichi, Tokyo 108-0014 (JP); TUSGITA, Akira, Tokyo 108-0014 (JP)
(74) Representative: Betten & Resch
(86) International application number: PCT/JP2007/052204
(87) International publication number: WO 2007/091627

(57) **Abstract**

The present invention is to provide a method for cleavage of peptidic bond at C terminal of peptide and a method for determination of C terminal amino acid sequence of peptide without the decrease of the sensitivity and with preventing the subsidiary reaction. The method for cleavage of peptidic bond at C terminal of peptide according to the present invention is **characterized** as follows:
A method for cleavage of peptidic bond at C terminal of peptide comprising the steps of:
reacting a peptide with a solution of perhalogenated carboxylic acid containing alcohol to esterify glutamic acid residue of said peptide; and
reacting said peptide with alkanoic acid anhydride to obtain C terminal-deleted peptide in which the amino acid residue of said peptide at C terminal is sequentially deleted. The method for determination of C terminal amino acid sequence of peptide according to the present invention is **characterized** as follows:
A method for determination of C terminal amino acid sequence of peptide comprising the steps of:
reacting a peptide with a solution of perhalogenated carboxylic acid containing alcohol to esterify glutamic acid residue of said peptide;
reacting said peptide with alkanoic acid anhydride to obtain C terminal-deleted peptide in which the amino acid residue of said peptide at C terminal is sequentially deleted;
measuring molecular weight of said C terminal-deleted peptide; and
grasping said peptide based on said molecular weight.

## Description

### Technical field

The present invention relates to a method for cleavage of peptidic bond at C terminal of peptide and a method for determination of C terminal amino acid sequence of peptide.

### Related art

Information related to the amino acid sequence of peptide or protein (hereinafter, collectively referred also to as peptide) obtained from natural origin is indispensable for researching the biological characterization and function. At present, the amino acid sequence of peptide and protein is determined as deduced amino acid sequence based on the corresponding gene information, that is, base sequence of genomic gene encoding these peptides or cDNA prepared from mRNA. Alternatively, these amino acid sequences of protein are determined by means of direct analysis of the sequence of peptide. In such case, knowledge of partial amino acid sequences of peptide is still necessary for identification of genomic gene encoding the peptide or cDNA prepared from mRNA.

Generally, it is considered that N terminal amino acid sequence and C terminal amino acid sequence of peptide are useful for the knowledge of the partial amino acid sequence of peptide. For example, in case of selection of cDNA encoding the peptide to be obtained from cDNA library prepared from numeral numbers of mRNA, if the N terminal amino acid sequence and C terminal amino acid sequence have already determined, it is possible to prepare a nucleic acid probe based on the amino acid sequence at both ends, and to select the cDNA to be obtained using the probe. In addition, it is possible to selectively amplify the cDNA to be obtained by utilizing PCR method with an oligonucleotide primer prepared from the basis of the amino acid sequence at both ends.

As the method for analyzing N terminal amino acid sequence of peptide, a method for determining the amino acid derivatives obtained from sequentially degrading the N terminal amino acid by Edman degradation method has been used.

On the other hand, as the method for analyzing C terminal amino acid sequence of peptide, a method for identifying C terminal amino acid has been proposed, in which C terminal amino acid is sequentially degraded by chemical reaction, and the degraded C terminal amino acid is determined based on the difference in molecular weight between the original peptide and the shortened peptide (Non-patent-related documents 1 to 3).

Non-patent-related document 1 discloses the method for sequentially degrading C terminal amino acid by chemical reaction. The method is a method for enhancing the selective hydrolysis of C terminal amino acid of peptide, in which the dry peptide is heated to 90°C, and is reacted with vapor created from high concentration of pentafluoropropanoic acid (CF₃CF₂COOH) aqueous solution or high concentration of heptafluorobutanoic acid (CF₃CF₂CF₂COOH) aqueous solution.

Non-patent-related documents 2 and 3 disclose the method for selectively degrading C terminal amino acid using acetonitrile solution of pentafluoropropanoic acid anhydride ((CF₃CF₂CO)₂O) or acetonitrile solution of heptafluorobutanoic acid anhydride ((CF₃CF₂CF₂CO)₂O), instead of high concentration of perfluoroalkanoic acid aqueous solution. The Documents state that the dry peptide is reacted with vapor created from the above-mentioned solution under -18°C, and the system is keep off the entry of water molecule vapored from the solution, thereby preventing to occur the subsidiary reaction.

It has been reported that these conventional methods for degrading C terminal amino acid are progressed by the dehydration reaction as stated in the following reaction formula (I) and the reaction as stated in the following reaction formula (II). That is, according to the reaction formula (I), a structure having the oxazolone ring will once be formed as the reaction intermediate from the C terminal amino acid. Next, it is considered to obtain the selective degradation reaction of the C terminal amino acid by acting the perfluoroalkanoic acid with the oxazolone ring.

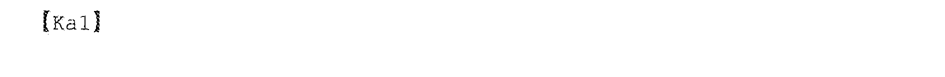

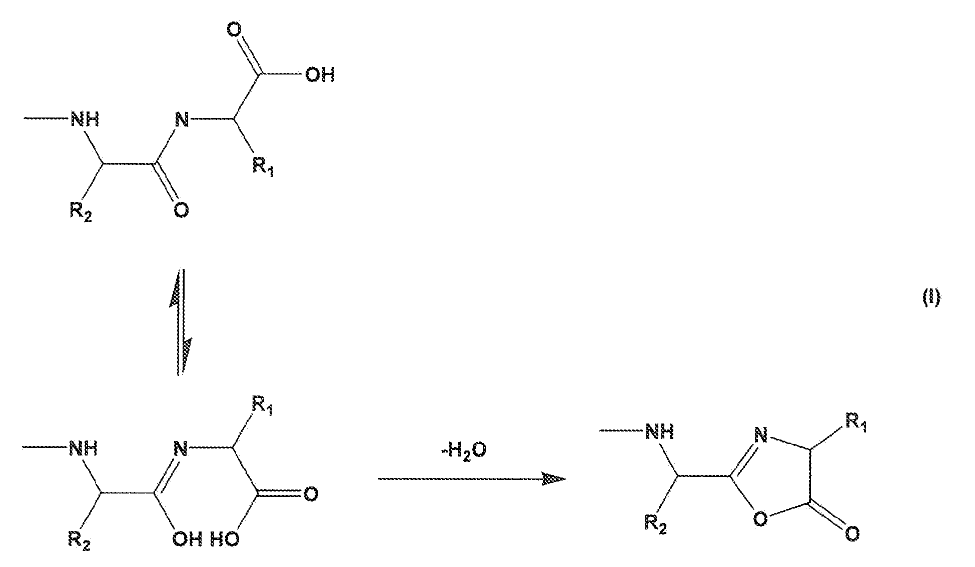

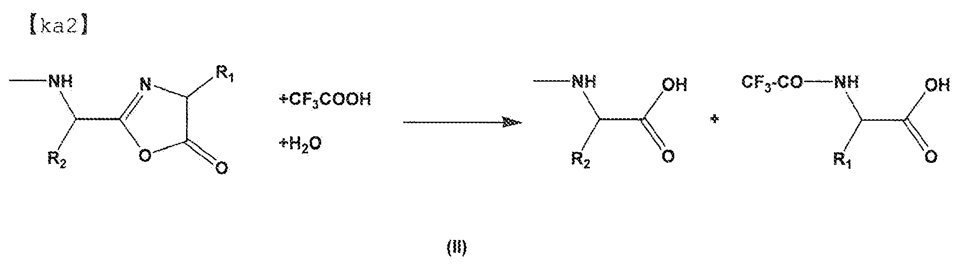

According to the method as disclosed in the Non-patent-related documents, cleavage of serine reside (-NH-CH(CH₂OH)-CO-) of the peptide at N terminal, cleavage of threonine residue at N terminal and cleavage of aspartate residue at C terminal have occurred in addition to the selective degradation of the target C terminal amino acid sequence. Therefore, there is a problem for selectively only degrading the C terminal amino acid sequence. In addition, due to N, O-acyl transfer reaction, which is a trigger for unintended cleavage, between an amino acid at α position of serine residue and hydroxyl group at β position, and between an amino acid at α position of threonine residue and β-hydroxyl group (β-OH group), the sequential degrading reaction of the C terminal amino acid sequence may be inhibited at the transfer reaction site.

In view of these cases, Patent document 1 discloses the method for sequentially degrading peptide under mild condition using an alkanoic acid anhydride such as acetic acid anhydride and formamide. However, in this reaction, there is a problem of low sensitivity.
[Patent document 1] WO 2005/078447
[Non-patent-related document 1] Tsugita, A. et al., Eur. J. Biochem., vol. 206, p. 691∼696, 1992
[Non-patent-related document 2] Tsugita, A. et al., Chem. Lett., p.235-238, 1992
[Non-patent-related document 3] Takamoto, K. et al., Eur. J. Biochem., vol. 228, p. 362-372, 1995

### Disclosure of Invention

### Problem to be solved in the present invention

The present invention is made in view of such problems. That is, it is confirmed that a new cleavage reaction provides the low sensitivity in determination of C terminal amino acid sequence by the conventional sequentially degrading method and the present invention is based on such a knowledge and property. The present invention is to provide a method for cleavage of peptidic bond at C terminal of peptide and a method for determination of C terminal amino acid sequence of peptide without the decrease of the sensitivity and with preventing the subsidiary reaction.

### Means for solving the problem

The method for cleavage of peptidic bond at C terminal of peptide according to the present invention is characterized as follows:
A method for cleavage of peptidic bond at C terminal of peptide comprising the steps of:
reacting a peptide with a solution of perhalogenated carboxylic acid containing alcohol to esterify glutamic acid residue of said peptide; and
reacting said peptide with alkanoic acid anhydride to obtain C terminal-deleted peptide in which the amino acid residue of said peptide at C terminal is sequentially deleted.

The method for determination of C terminal amino acid sequence of peptide according to the present invention is characterized as follows:
A method for determination of C terminal amino acid sequence of peptide comprising the steps of:
reacting a peptide with a solution of perhalogenated carboxylic acid containing alcohol to esterify glutamic acid residue of said peptide;
reacting said peptide with alkanoic acid anhydride to obtain C terminal-deleted peptide in which the amino acid residue of said peptide at C terminal is sequentially deleted;
measuring molecular weight of said C terminal-deleted peptide; and
grasping said peptide based on said molecular weight.

In addition, the method for determination of C terminal amino acid sequence of peptide according to the present invention is characterized as follows:
A method for determination of C terminal amino acid sequence of peptide comprising the steps of:
reacting a peptide with a solution of perhalogenated carboxylic acid containing alcohol to esterify glutamic acid residue of said peptide;
reacting said peptide with alkanoic acid anhydride to obtain C terminal-deleted peptide in which the amino acid residue of said peptide at C terminal is sequentially deleted;
reacting said peptide with site specific cleaving agent to obtain a C terminal-deleted peptide originated peptide fragment originated from said C terminal-deleted peptide;
measuring molecular weight of said C terminal-deleted peptide originated peptide fragment; and
grasping said peptide based on said molecular weight.

The method for determination of C terminal amino acid sequence of peptide supported in a substrate according to the present invention is characterized as follows:
A method for determination of C terminal amino acid sequence of peptide supported in a substrate comprising the steps of:
reacting a peptide supported in a substrate with a solution of perhalogenated carboxylic acid containing alcohol to esterify glutamic acid residue of said peptide;
reacting said peptide with alkanoic acid anhydride to obtain C terminal-deleted peptide in which the amino acid residue of said peptide at C terminal is sequentially deleted;
immersing said substrate into a solution containing site specific cleaving agent to obtain a C terminal-deleted peptide originated peptide fragment originated from said C terminal-deleted peptide;
measuring molecular weight of said C terminal-deleted peptide originated peptide fragment; and
grasping said peptide based on said molecular weight.

### Effect of Invention

According to the present invention, the cleavage of the glutamic acid residue of the peptide at N terminal by sequentially degrading peptidic bond at C terminal is suppressed by specific esterification of glutamic acid residue, thereby specifically and effectively degrading C terminal amino acid residue. In addition, assignment of the C terminal-deleted peptide as obtained in sequential degradation and of the peptide originated from the C terminal-deleted peptide is easily made.

### Brief Explanation of the Drawing

Figure 1 is a figure showing the amino acid sequence of soybean trypsin inhibitor and the deduced sequence of digested fragment as digested with trypsin.
Figure 2 is mass analysis spectrum of the peptide fragment at 48 to 63 residues of trypsin inhibitor.
Figure 3 is mass analysis spectrum of the peptide fragment at 112 to 119 residues of trypsin inhibitor.
Figure 4 is mass analysis spectrum of the peptide fragment at 66 to 76 residues of trypsin inhibitor.
Figure 5 is mass analysis spectrum of the peptide fragment at 31 to 38 residues of trypsin inhibitor.
Figure 6 is mass analysis spectrums showing an influence for the esterification reaction to the method for analyzing according to the present invention, wherein (A) corresponds to the result without the esterification reaction and (3) corresponds to the result with the esterification reaction.
Figure 7 is mass analysis spectrum showing an influence for the esterification reaction to the method for analyzing according to the present invention.
Figure 8 is mass analysis spectrum of the peptide fragment at 49 to 63 residues of trypsin inhibitor.

### Best mode for carrying out the present invention

### <Introduction - New cleavage reaction and mode by sequential degradation>

In the connection with the present invention, the present inventors have found that an unknown and specific cleavage reaction has occurred in glutamic acid residue at N terminal during sequential cleavage of peptide under mild condition using an alkanoic acid anhydride such as acetic acid anhydride and formamide. In addition, the present inventors have found that this cleavage mode is absolutely new. These knowledge will be indicated as follows.

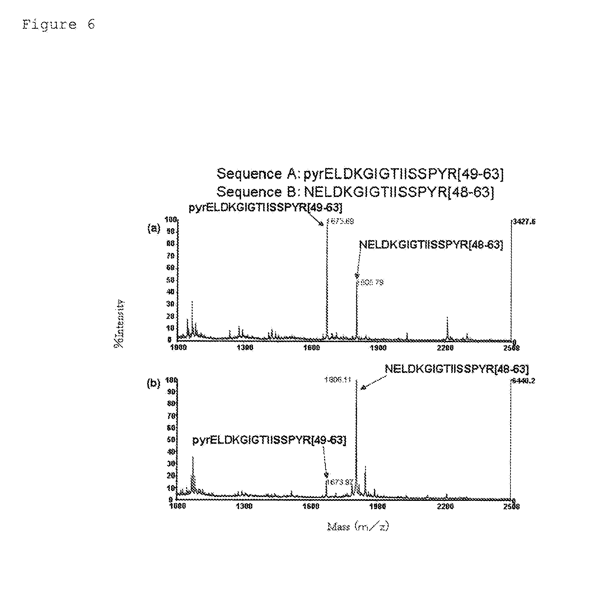

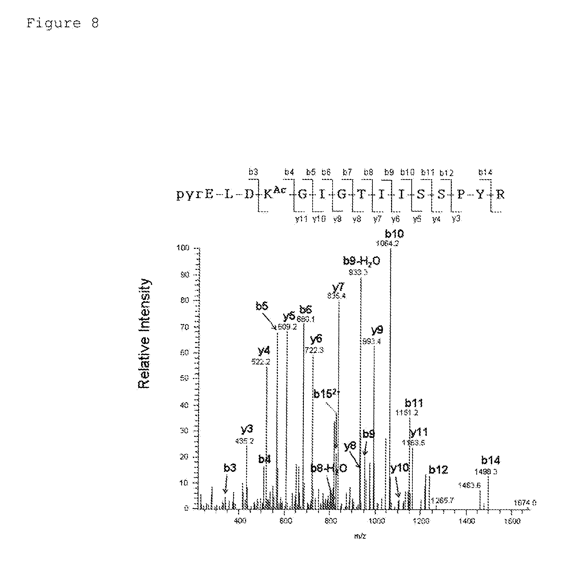

Figure 6 (a) is MALDI-TOF-MS spectrum of an eluted mixture wherein the gel containing soybean trypsin inhibitor after performing SDS-PAGE is immersed in a solution containing acetic acid anhydride and formamide at 60°C for 1 hour, and the gel is digested with trypsin to obtain the eluted mixture from the gel. As shown in Figure 6 (a), in case of trypsin digestion of trypsin inhibitor, in addition to the peak corresponding to the sequence from 48 to 63 residues containing aspartate residue at 48 redisue which is theoretically considered as being cleaved by trypsin digestion after the treatment of sequential cleavage, the other peak was found around 1673.69. It is supposed that this peak is a peak corresponding to the peptide fragment containing glutamic acid residue at 49 residue of the trypsin inhibitor, that is, the peptide fragment at 49 to 63 residues (sequence number 26) based on the molecular weight thereof. However, it has found that molecular weight of this peak is less about 18 for identifying to contain glutamic acid residue in the peak at 1673.69, even though the peak is compared with the peak corresponding to the peptide fragment of 48 to 63 residues. Consequently, the result of MS/MS analysis for this fragment in order to determine the origin of this peak is shown in Figure 8. As clearly shown in Figure 8, there is found a peak at the mass position less than 18 from the theoretical molecular weight in the fragment containing the amino acid residue at N terminal, while there are found the peaks in theoretical mass in the group of fragments containing arginine (R indicated as One character) at C terminal (y3 to y11).

As summarized these results, the followings have found that:
(1) specific cleavage reaction at N terminal of glutamic acid residue has occurred in the conventional sequential cleavage of peptide using alkanoic acid anhydride and formamide; and
(2) the fragment as obtained in this cleavage reaction is a fragment having 18 less in molecular weight than that of predicted amino acid residue.

These results strongly indicate that, among the peptide fragments obtained in the above-mentioned sequential cleavage, the peptide fragment containing glutamic acid residue at N terminal is dehydrated. In addition, it is strongly suggested that the glutamic acid residue is transferred to the pyroglutamic acid residue as molecular family created from the dehydration of the glutamic acid residue. This cleavage reaction and cleavage mode are absolutely new which has not found in the conventional sequential cleavage reaction. The present invention is to provide the method for cleavage of peptidic bond at C terminal of peptide and the method for determination of C terminal amino acid sequence of peptide without the decrease of the sensitivity and with effectively preventing the subsidiary reaction, based on the above-mentioned knowledge.

It should be noted that, among the peptide fragment created from the sequential cleavage reaction, a peptide fragment into which the peptide fragment containing glutamic acid residue is hydrated refers to as a hydrated body, and that the hydrated body of the peptide fragment is indicated in Figures as a name having an abbreviation "*pyr*" at beginning.

### <One aspect of the method for cleavage of peptidic bond at C terminal of peptide according to the present invention>

The method for cleavage of peptidic bond at C terminal of peptide according to the present invention is characterized in that:
A method for cleavage of peptidic bond at C terminal of peptide comprising the steps of:
reacting a peptide with a solution of perhalogenated carboxylic acid containing alcohol to esterify glutamic acid residue of said peptide; and
reacting said peptide with alkanoic acid anhydride to obtain C terminal-deleted peptide in which the amino acid residue of said peptide at C terminal is sequentially deleted. Hereinafter, the steps are referred to as an esterification step and a cleavage step, respectively.

### (The esterification step)

The esterification step may be performed in a solution or in a substrate. In case of performing the esterification step in the substrate supporting the peptide, the substrate is not limited so far as it is a composition which can hold the peptide. For example, the substrate includes polyacrylamide gel. The substrate may be any substrates used for, of course, the conventional SDS-PAGE to electrophore in the direction of first dimension, and for the two dimensional electrophoresis.

The alcohol is not limited so far as it is a compound having alcoholic hydroxyl group capable of esterification reaction with an amino acid residue. For example, the alcohol may be alcohol having 1 to 5 carbon atoms. The example of the alcohol includes lower alcohol such as methanol, ethanol, propanol, butanol and pentanol. Especially, methanol is more preferable. In addition, the other atom(s) than the hydroxyl group of the alcohol is not limited in any one having various masses, so far as the other atom does not inhibit the esterification reaction of the amino acid residue with the alcohol.

Any peptides can be used as the peptide in the present invention. The peptide may have intramolecular/intermolecular binding such as S-S bonding, have any coordination metals, be changed in the side chain on dependence on the redox condition, and be a peptide subjected to post translational modification with phosphate, sugar chain and the other. In addition, in case of performing the step in the substrate, the peptide is not limited so far as it can be held in the substrate.

The perhalogenated carboxylic acid may be any carboxylic acids having halogen atom such as fluorine, chlorine and bromine. The perhalogenated carboxylic acid may have lower carbon chain. The example of the perhalogenated carboxylic acid includes trichloroacetic acid, trifluoroacetic acid, pentafluoropropionic acid and heptafluorobutyric acid. Especially, trifluoroacetic acid is preferable in view of permeability it into the substrate.

In the present invention, the solution of perhalogenated carboxylic acid containing the alcohol may contain a solvent capable of keeping the alcohol and the perhalogenated carboxylic acid in liquid state. For example, the solvent for the solution of the perhalogenated carboxylic acid includes water and alcohol. That is, the solution of perhalogenated carboxylic acid containing the alcohol may be aqueous solution and alcohol solution.

Temperature, time, concentration and pH of performing the esterification step may be appropriately adjusted.

The temperature is not limited so far as the peptide is not eluted from the substrate and the liquid phase can be kept in liquid state. For example, the temperature is preferable in the range of 4 to 40°C, more preferable in the range of 20 to 30°C, and especially preferable in the range from room temperature to 25°C. In case of less than 0°C, the speed of the esterification will become low, and in case of more than 40°C, subsidiary reaction such as a cleavage reaction of the peptide with perhalogenated carboxylic acid will be occurred.

The reacting time may be appropriately changed depending on the temperature for the reaction. The time may be about 6 hours in dependence on the temperature. In case of less than 120 minutes, the esterification reaction of the glutamic acid residue with the perhalogenated carboxylic acid will not be sufficiently in progress, in case of more than 2 days, the background will become high due to the oxidation and elution of the sample, thereby declining the quality of the signal.

Concentrations of each composition of the solution of the perhalogenated carboxylic acid containing the alcohol used in the esterification step are not limited. The concentration of the alcohol in the solution is preferably in the range of 5 to 30 v/v%. The concentration of the perhalogenated carboxylic acid in the solution is preferably 10 to 40 v/v%. In case of less than 1 v/v% of the alcohol, the alcohol which will be supplied to glutamic acid will be consumed for reaction with acids, thereby insufficiency of the alcohol, and in case of more than 60 v/v%, the gel is dehydrated and shrinked thereby avoiding the reaction. In case of less than 1 v/v% of the perhalogenated carboxylic acid, acids will be consumed for the esterification reaction with the alcohol, thereby shortage of catalysis, and in case of more than 60 v/v%, unnecessary dehydration will be occurred thereby leading to adverse effect. When methanol is used as the alcohol and trifluoroacetic acid is used as the perhalogenated carboxylic acid, the ratio of water, methanol and trifluoroacetic acid in the solution of the perhalogenated carboxylic acid containing the alcohol is preferably in the range of 100∼600:150∼350:E300∼700, and more preferable in the range of 100:50:100∼500:250:500.

When the peptide as held in the substrate is esterified, it is possible to enhance delivery of the reagent to the peptide by which after the substrate is intentionally dehydrated and shrinked with the alcohol so as to increase the yield of the reaction, the gel is immersed in the alcohol solution of the perhalogenated carboxylic acid to swell the substrate by utilizing water created from the reaction of the perhalogenated carboxylic acid with the alcohol. For example, when methanol is used as the alcohol and TFA is used as the perhalogenated carboxylic acid, the substrate which is dehydrated and shrinked with methanol may be applied in the solution containing methanol and TFA with the ratio of methanol and TFA of 250:500, whereby obtaining the swollen gel under the mild condition for several hours at room temperature, and progress in the specific esterification reaction of glutamic acid.

In the esterification step, carboxyl group of glutamic acid residue of the peptide as supported in the substrate will be mainly transferred into an esterified derivative corresponding to the alcohol used in the above-mentioned reaction (e.g. in case of methanol, methylcarboxyl group).

### (The cleavage step)

In the cleavage step, the peptidic bond of the peptide is sequentially cleaved using the alkanoic acid anhydride. The peptidic bond of the peptide at C terminal is sequentially cleaved by reacting the peptide to be cleaved with the alkanoic acid anhydride to obtain a C terminal-deleted peptide in which the amino acid residue is sequentially deleted from the C terminal. The alkanoic acid anhydride to be used in the cleavage step includes substituted or non-substituted alkanoic acid anhydride, but does not include perfuloroalkanoic acid and anhydride thereof. Meanwhile, in case of using the substituted alkanoic acid anhydride, it is preferable to use the alkanoic acid anhydride substituted with any atoms other than halogen. A symmetrical acid anhydride of alkanoic acid having about 2 to 6 carbon atoms may be used as the alkanoic acid anhydride used for the activation of carboxyl group at C terminal of the peptide. By selecting such an aspect, an appropriate reactivity can be ensured in case of increasing to the reaction temperature. In addition, a symmetrical acid anhydride of alkanoic acid having about 2 to 4 carbon atoms may be preferably used. By selecting such an aspect, a steric hindrance can be reduced. In addition, a symmetrical acid anhydride of linear alkanoic acid having about 2 to 6 carbon atoms may be used as the acid anhydride. In addition, a symmetrical acid anhydride of linear alkanoic acid having about 2 to 4 carbon atoms may be preferably used. By selecting such an aspect, a steric hindrance can be reduced. In particular, a symmetrical acid anhydride of linear alkanoic acid having 2 atoms, that is, acetic acid anhydride, may be used.

In addition, in order to try to activate carboxyl group at C terminal and to appropriately arrange for forming 5-oxazolone ring, it is preferable that a compound which less occur the steric hindrance may be used as the alkanoic acid anhydride. On this regard, acetic acid anhydride is preferably used.

It should be noted to desire that extremely excess quantity of the alkanoic acid anhydride is preliminarily solved in the bipolar aprotic solvent which is utilized for swelling the substrate to suppress the decrease of the concentration, since the alkanoic acid anhydride will be consumed along with the progress of the reaction. The concentration of the alkanoic acid anhydride in the reaction solution may be in the range of 1% by volume to 30% by volume, preferably in the range of 10% by volume to 20% by volume. In addition, the reaction temperature may be greater than or equal to 50°C, or preferably greater than or equal to 60°C. By selecting such an aspect, the sequential cleavage can be effectively performed. In addition, the reaction temperature may be less than or equal to 100°C, or preferably less than or equal to 80°C. By selecting such an aspect, the sequential cleavage can be stably performed. In addition, it may be reacted for about 4 to 110 hours in case of using acetic acid anhydride. In such case, the reaction condition may be set as at 50°C for 110 hours, at 60°C for 50 to 60 hours, at 80°C for 24 hours or at 100°C for 4 hours. The reaction condition can be set as the mild condition along with lowering the reaction temperature, thereby further suppressing to occur the subsidiary reaction.

On the other hand, in the cleavage step, the alkanoic acid anhydride may be solved in the bipolar aprotic solvent such as formamide. As the bipolar aprotic solvent, a solvent capable of infiltrating into the substrate at the temperature from about 50 to 90°C and of keeping the substrate in the swelling state is used. In addition, an organic solvent having relatively small molecular size and superior to the affinity to the material of the substrate is used. In addition, the bipolar aprotic solvent may be a solvent which indicates high bipolarity capable of sustaining the ratio of enol body during the compatible isomerization process as stated in the Formula (I) as mentioned above and is superior to the alkanoic acid anhydride solute and the alkanoic acid of the by-product of the reaction. In addition, the bipolar aprotic solvent is preferably to less volatile and evapolate at the reaction temperature.

The cleavage step may be performed in the dry atmosphere in which oxygen is removed. The method for creating such an atmosphere includes, for example, a method in which the system to be performed the reaction is set as airtight state, entry of moisture and oxygen from outside the system is prevented, introduction and discharge of any liquids used for the reaction is performed under inert gas atmosphere such as dry nitrogen and argon. In addition, oxygenation may be avoided using compound having reducing sulfanil group (-SH) such as DTT having effect of antioxydation. By removing oxygen, oxidation of sulfur of methionine residue is prevented thereby improving accuracy of determining the molecular weight in the measurement step.

A reaction accelerator may be used in the cleavage step. The accelerator includes basic aromatic ring compound containing nitrogen atom, and may be pyridine base or derivative thereof. The pyridine base acts as a proton acceptor. Therefore, it can rapidly remove proton to be withdrawn accompanied by acylation to the amino group. In particular, the pyridine base includes, for example, pyridine, picoline (methyl pyridine), lutidine (dimethyl pyridine), collidine (trimethyl pyridine), ethyl methyl pyridine and parvoline (tetramethyl pyridine). The basic aromatic ring compound containing nitrogen atom includes condensed ring aza arene. Bicyclic basic aromatic ring compound containing nitrogen atom such as quinoline, isoquinoline and indole, or derivatives of the compound may be used as the basic aromatic ring compound containing nitrogen atom. In addition, as the basic aromatic ring compound containing nitrogen atom, tricyclic basic aromatic ring compound containing nitrogen atom or derivative of the compound such as azaanthracene and phenanthridine including benzoquinoline, benzoisoquinoline and acrydine.

In case of using pyridine as the reaction accelerator, the concentration is in the range of 1 v/v% to 40 v/v%, preferably in the range of 10 v/v% to 30 v/v%, thereby securely increasing the speed of the sequential cleavage at C terminal. In addition, when the substrate is immersed in this solution, immersion of the substrate for 20 minutes at room temperature may be performed three times. Then, the sequential cleavage reaction with acid anhydride may be attained by removing the excess catalyst and moisture from the substrate using the polar aprotic solvent.

It should be noted that the reaction in the cleavage step may be stopped by diluting and removing the reactive agent alkanoic acid anhydride infiltrated in the substrate with such as water, bipolar aprotic solvent and polar aprotic solvent, along with lowering the temperature of the reaction system. In such case, the polar aprotic solvent which does not solve the substrate material and which has affinity to alkanoic acid anhydride and bipolar aprotic solvent may be used. In case of using polyacrylamide gel, the polar aprotic solvent includes nitriles having less than or equal to 4 carbon atoms such as acetonitrile, and ketones having less than or equal to 4 carbon atoms such as acetone.

Then, as mentioned above, 5-oxazolone ring which is formed by reacting the peptide with alkanoic acid anhydride is hydrolyzed to form a compound having carboxyl group end. This hydrolysis reaction is not limited so far as it is any well-known method, and basic aromatic ring compound containing nitrogen atom or tertially amines may be used as catalyst. A monocyclic aromatic ring compound containing nitrogen atom indicating high solubility to the polar aprotic solvent may be used as the basic aromatic ring compound containing nitrogen atom. In particular, pyridine or pyridine base and the other are preferable. In addition, as the tertially amines, a compound which indicates the same relatively weak basicity as that of pyridine base is used. In particular, DMAE (dimethylamine ethanol ((CH₃)₂N-CH₂CH₂OH) is preferable as the tertially amines. In case of using pyridine as the basic aromatic ring compound containing nitrogen atom, the content may be in the range of 5% by volume to 15% by volume to the total volume of the aqueous solution, in particular, 10% by volume. In addition, in case of using DMAE, the content may be in the range of 1% by volume to 20% by volume to the total volume of the aqueous solution, in particular, 10% by volume. In addition, the hydrolysis reaction may be performed at more than or equal to 50°C. In case of performing the reaction in airtight reaction container, the temperature may be at less than or equal to 100°C.

Further, the hydrolysis reaction may be progressed along with the reaction forming 5-oxazolone ring with the alkanoic acid anhydride. In such a case, an aqueous solution containing an organic base is added along with decreasing the reaction temperature of the sequential cleavage reaction of the peptidic bond at C terminal to stop the reaction. By selecting such an aspect, inactivation of the alkanoic acid anhydride and elution from the substrate will be occurred. Consequently, the sequential cleavage reaction of the peptidic bond at C terminal will be stopped, and the reaction agent will be inactivated and removed. Then, the hydrolysis treatment to the reaction product is performed, and finally, dehydrating treatment is again performed with the polar aprotic solvent. By selecting such an aspect, it will be occurred the removal of alkanoic acid corresponding to alkanoic acid anhydride and of bipolar aprotic solvent, along with an aqueous solution containing organic base, and re-dehydration will be occurred. Consequently, a treatment which does not substantially differ from a treatment comprising washing and removal step with polar aprotic solvent at the middle of the treatment can be easily performed.

By the method for cleavage of peptidic bond at C terminal of peptide according to the present invention, specific esterification reaction is progressed in the esterification step to obtain the esterified derivative (for example, a compound containing methyl group) corresponding to alcohol (for example, methanol in the esterification step. Next, peptidic bond of the peptide at C terminal is sequentially cleaved in the cleavage step. The sequential cleavage reaction at C terminal is specifically progressed without cleaving the bond at N terminal of glutamic acid residue, since glutamic acid residue is esterified. Therefore, the present invention can be applied in various applications including mass analysis to keep the quantitative capability of the reaction product without lowering the sensitivity, since the sequential cleavage reaction is progressed in the state of being supported the peptide in the substrate without eluting the peptide to be cleaved from the substrate.

### <One aspect of the method for determination of C terminal amino acid sequence of peptide according to the present invention>

The method for determination of C terminal amino acid sequence of peptide according to the present invention is characterized as follows:
A method for determination of C terminal amino acid sequence of peptide comprising the steps of :
reacting a peptide with a solution of perhalogenated carboxylic acid containing alcohol to esterify glutamic acid residue of said peptide;
reacting said peptide with alkanoic acid anhydride to obtain C terminal-deleted peptide in which the amino acid residue of said peptide at C terminal is sequentially deleted;
measuring molecular weight of said C terminal-deleted peptide; and
grasping said peptide based on said molecular weight. The aspect further comprises steps of measuring a molecular weight of a C terminal-deleted peptide and of grasping the peptide based on the molecular weight along with the above-mentioned esterification step and cleavage step. These steps are referred to as measurement step and grasp step, respectively. In this aspect, the esterification step and cleavage step may be performed in the manner as mentioned above, in addition to the manner as mentioned below. This aspect can be applied in any peptides having various molecular weights so far as the peptide is supported in the substrate. This aspect can be preferably used in case of determination of the peptide having relatively low molecular weight as C terminal amino acid sequence to be determined. The peptide may have less than 5000 of molecular weight. In addition, in this aspect, the digestion step using site specific cleavage reagent as mentioned below may be performed between the cleavage step and the measurement step.

### (The measurement step)

The measurement step is a step for measuring a molecular weight of peptides such as C terminal-deleted peptide. Various measuring means of the molecular weight may be used in performing this step. The means include mass analysis apparatus. For example, ion trap mass spectrometer, quadrupole mass spectrometer, magnetic sector-type mass spectrometer, time-of-flight mass spectrometer and Fourier transform mass spectrometer may be used as the mass analysis apparatus. In addition, the ionization method includes electrospray ionization (ESI method), matrix-assisted laser desorption/ionization (MALDI) method and fast atom bombardment ionization (FAB) method).

Among these, MALDI-TOF-MS is, for example, preferably used. By using MALDI-TOF-MS, lack of part (s) of atomic family from amino acid residue constituting the peptide fragment can be suppressed during the ionization process. In addition, it can preferably perform the measurement of relatively high molecular weight of the peptide fragment. Furthermore, even in case of electrophoring the peptide to be measured in the substrate, performing the above-mentioned treatment in the substrate, and then collecting and provided to the measurement, both of the corresponding anionic ion and cationic ion can be measured. For such reasons, high reproducibility of analysis can further be performed by using MALDI-TOF-MS.

In case of using MALDI-TOF-MS in the measurement step, cationic species adduced proton (H⁺) to the peptide fragment and anionic species withdrawn proton from the peptide fragment may be measured in the ionization of MALDI-TOF-MS, respectively.

It should be noted that length of the peptide fragment provided to mass analysis in the measurement step may be in the range of 30 to 50 amino acid residues at most, and 20 to 30 amino acid residues. Consequently, the peptide fragment can be surely ionized, and the measurement can be performed with high accuracy. The molecular weight of the peptide may be in the range not greater than 4000, and preferably in the range not greater than 3000. Consequently, in case of identifying the corresponding amino acid based on difference of the molecular weight, such as asparagine residue (Asn) and aspartate residue (Asp), and glutamine residue (Gln) and glutamate residue (Glu), discrimination of amino acid residue having 1 difference in the chemical formula weight can be performed with high accuracy.

### (The grasp step)

The grasp step is a step of grasping the peptide based on the molecular weight such as C terminal-deleted peptide obtained in the measurement step and the original peptide. This step may be performed such that the C terminal amino acid sequence of the original peptide is grasped to compare the difference between the molecular weight of C terminal-deleted peptide having various lengths obtained from the sequential cleavage as mentioned above, and the molecular weight of the original peptide.

In the method for determination of C terminal amino acid sequence of peptide according to the present invention, the sequential cleavage will be specifically progressed in the C terminal amino acid sequence, since the cleavage reaction of glutamic acid residue at N terminal is suppressed. The obtained C terminal-deleted peptide will be a peptide stepwisely deleted amino acid residue at C terminal by the sequential cleavage without being unspecifically cleaved in the amino acid residue constituting the peptide other than at C terminal. Therefore, the C terminal amino acid sequence can be grasped based on the difference in molecular weight between the C terminal-deleted peptide and the original peptide, without consideration of other possibility.

### <Other aspect of the method for determination of C terminal amino acid sequence of peptide according to the present invention>

The other aspect of the method for determination of C terminal amino acid sequence of peptide according to the present invention is characterized as follows:
A method for determination of C terminal amino acid sequence of peptide supported in a substrate comprising the steps of:
reacting a peptide supported in a substrate with a solution of perhalogenated carboxylic acid containing alcohol to esterify glutamic acid residue of said peptide;
reacting said peptide with alkanoic acid anhydride to obtain C terminal-deleted peptide in which the amino acid residue of said peptide at C terminal is sequentially deleted;
immersing said substrate into a solution containing site specific cleaving agent to obtain a C terminal-deleted peptide originated peptide fragment originated from said C terminal-deleted peptide;
measuring molecular weight of said C terminal-deleted peptide originated peptide fragment; and
grasping said peptide based on said molecular weight. This aspect further comprises the digestion step between the cleavage step and measurement step along with the above-mentioned esterification step, cleavage step, measurement step, and grasp step. This aspect is preferably used in case of determining C terminal amino acid sequence of the peptide having relatively high molecular weight. The molecular weight may be, for example, in the range of 5 kDa to 300 kDa. The esterification step, cleavage step, measurement step and grasp step in this aspect may be performed in the manner as mentioned above, in addition to the manner as mentioned below.

### (The esterification step)

The esterification step of the peptide supported in the substrate is a method for reacting with the perhalogenated carboxylic acid containing alcohol as mentioned above. In addition, a method for attain the swelling of the substrate and the infiltration of the reagent may be used as the esterification step wherein the method comprises the steps of reacting an alcohol solution of perhalogenated carboxylic acid to the substrate dehydrated and shrinked with alcohol so as to utilize water created from the reaction of perhalogenated carboxylic acid and alcohol. By the method, it is possible to attain more rapid esterification reaction.

### (The digestion step)

The digestion step is a step of immersing the substrate supporting the peptide into a solution containing site specific cleavage reagent such as proteolytic enzyme. The step provides various peptide fragments. It selectively performs the peptide chain at binding site of predetermined amino acid residue with regard to mixture containing a series of reaction product in which a predetermined number of amino acid residue is deleted from C terminal of the original peptide, after performing the above-mentioned method for cleavage of peptidic bond at C terminal of peptide. In this case, enzymatic digestion of relatively high molecular weight peptide chain may be performed using site specific protease such as trypsin. The site specific cleavage reagent used in the digestion step may be other proteases having site specificity such as V8 protease which cleaves glutamic acid residue at C terminal, in addition to trypsin. In addition, any chemical reagent which specific cleaves amide bonding of methionine at C terminal such as CNBr may be used.

The digestion step is performed in the state of supporting the peptide in the substrate. The peptides and/or the peptide fragments will be eluted into the liquid phase used in the digestion step, when the peptide or peptide fragment becomes to have a short length which can not be supported in the substrate. The mixture of the peptide fragments as obtained from the digestion step at least contains peptide fragments at C terminal originated from the original peptide and peptide fragments at C terminal originated from the series of reaction product in which the predetermined number of amino acid residue at C terminal is deleted.

### (The measurement step)

The peptides obtained in this aspect contain the peptide fragment originated from the original peptide, peptide, fragment originated from the C terminal-deleted peptide and peptide fragment at N terminal side, in addition to the original peptide and C terminal-deleted peptide. Therefore, the versatility can be more improved, by selecting an approach capable of accurately determining each molecular weight as observed in the peak originated from the original peptide and the peptide fragment at C terminal from the series of reaction product, upon discriminating the peak from peptide fragment at C terminal originated from the original peptide and the series of reaction product with the other peak from the plurality of the peptide fragments at N terminal with high accuracy.

Here, in case of using trypsin in the digestion step, the amino acid reside constituting the peptide fragment at C terminal does not contain arginine residue, since the step is performed after the digestion step. On the other hand, the other peptide fragment obtained in the digestion step will contain arginine residue having guanidine group which is rich in proton acceptability. Consequently, the cationic ion originated from these components will be stabilized. As such, in the measurement step, the measurement condition may be appropriately selected in accordance with cleavage mode of the peptide with the site specific cleavage reagent used in the digestion step.

Consequently, in the mass analysis, the relative intensity between the peptide fragment at C terminal and the other peptide fragment is different from each other in comparison with the result from the cationic species and the anionic species. By utilizing such phenomenon, the peaks derived from the series of peptide fragments at C terminal may be discriminated and identified among the several types of the peaks as measure by MALDI-TOF-MS apparatus.

The peak intensity derived from the peptide fragments containing arginine residue at C terminal is relatively high in the mass analysis spectrum of the cationic species. On the other hand, the peptide fragment at C terminal not containing arginine residue contains carboxyl group which indicates proton-donating ability at C terminal. Consequently, the peak intensity derived from the peptide fragment at C terminal is relatively high in the mass analysis spectrum of the anionic species measured by MALDI-TOF-MS apparatus. As such, the peaks originated from peptide fragments commonly containing arginine residue at C terminal side may be discriminated by utilizing the difference of the relative intensities in comparison of the mass analysis spectrum of cationic species in MALDI-TOF-MS with the mass analysis spectrum of anionic species. In addition, the peak of the original peptide chain and the peak originated from the series of the peptide fragments at C terminal obtained in the sequential cleavage reaction of the C terminal amino acid residue may be easily identified.

### <Further step>

The above-mentioned method for cleavage of peptidic bond at C terminal of peptide and the method for determination of C terminal amino acid sequence of peptide according to the present invention may further comprise various pre-treatment and post-treatment, in addition to the above-mentioned steps.

### (Dehydration of the substrate)

The substrate may be dehydrated with a polar aprotic solvent which does not solve any components constituting the substrate such as gel-like material, and has an affinity to water. By using this method, the peptide to be analyzed can be held in the substrate as separated spot or band even after the dehydration treatment is finished. In case of polyacrylamide as the substrate, nitriles having less than or equal to 4 carbon atoms such as acetonitrile which is rich in affinity to water, ketones having less than or equal to 4 carbon atoms such as acetone may be used as the polar aprotic solvent used for the dehydration. In addition, these polar aprotic solvent are easily evaporated than water. When the polar aprotic solvent is evaporated and the substrate is dried, the bulk volume of the substrate will decrease and the substrate will be shrinked. Immersion of the substrate is repeatedly performed in several times. For example, the substrate piece may be immersed three times for 20 minutes in room temperature.

### (Crosslinkage of the peptide)

The peptide supported in the substrate may be crosslinked each other prior to performing the esterification step. The crosslinker is not limited so far as it has bonding group at its ends. The crosslinker may have aldehyde group such as formaldehyde and glutaraldehyde. Consequently, the peptides are crosslinked in the substrate supporting the peptide to form network structure, thereby entangling the substrate and the peptide, and suppressing the elution of the peptide. In case of using glutaraldehyde as the crosslinker, the crosslinkage of the peptide may be performed by immersing the substrate into glutaraldehyde aqueous solution having a concentration of 1 pmol/µL to 1000 nmol/µL for 30 minutes to 2 hours.

### (Treatment for Intermolecular/intramolecular binding and post-translational modification)

In the present invention, molecular family of the intermolecular/intramolecular biding and of the post-translational modification of the peptide to be objected may be subjected to a treatment such as degradation and cleavage with an appropriate treating agent. For example, in case of peptide intermolecularly or intramolecularly having S-S bonding to be objected, S-S bonding may be cleaved by reducing it with reducing agent. In addition, SH group obtained by reduction of S-S bonding may be treated further with alkylating agent to prevent the re-formation of S-S bounding.

### (Protection of N or O atom)

In the present invention, β-hydroxyl group (β-OH group) such as serine residue and threonine residue and or ε-amino group (ε-NH₂ group) such as lysine residue may be protected for the purpose of preventing the possible N,O-acyl transfer reaction during the sequential cleavage of the peptide in the above-mentioned cleavage step and a cleavage reaction followed by the transfer reaction. In addition, ε-NH₂ group may be protected so as to uniform the sensitivity of lysine residue to trypsin since the natural origin dimethyl and acetyl derivatives of lysine residue is not subjected to cleavage with trypsin, one of proteases. In this case, lysine residue will be transferred into the protected substituted group as protected in ε-NH₂ group along with the natural origin dimethyl and acetyl derivatives, thereby hard to occur the cleavage of lysine residue at N terminal side by trypsin. These protections are not limited so far as using with the well-know protective group for hydroxyl group and amino group. For example, the protection may be performed with O-acylation and N-acylation. For example, the above-mentioned acylation may be performed such that the substrate is stirred in a mixture of alkanoic acid anhydride as added small amount of alkanoic acid. In the case, alkanoic acid anhydride corresponds to the electrophilic acylating agent. In addition, alkanoic acid enhances the reactivity of alkanoic acid anhydride with amino group and hydroxyl group due to proton-donating ability, thereby occuring N-acylation and O-acylation. The acylation may be stopped in accordance with the stoppage of the reaction performed in the cleavage step.

### (Post-treatment)

Desolvation may be performed after the cleavage step. The desolvation treatment is not limited, and may be performed in accordance with dehydration of the substrate as mentioned above.

Prior to the measurement step, the mixture containing the peptides and/or peptide fragments to be measured may be deminelized. Consequently, the peptide fragment to be collected and dried does not form a salt thereof, and form a primary peptide.

Furthermore, selective cleavage reaction of amino acid residue at C terminal of the peptide chain is progressed along with an acetylation by reacting the peptide chain at C terminal held in the re-swelling substrate with acetic acid anhydride at heating temperature. In particular, it is supposed to make progress, at C terminal of the peptide, the sequential cleavage reaction of the C terminal amino acid of the peptide chain via the formation of 5-oxazolone ring through the reaction pathway as mentioned in Formulas (I) and (II).

In case of making progress the sequential cleavage reaction of C terminal amino acid, a mixture containing a series of reactive product which is stepwisely deleted C terminal amino acid and the original peptide as protected by acetylation will be remained in the substrate.

Each aspect of the present invention is explained on detail as mentioned above. These aspects are explained with an example. A person skilled in the art can appreciate that several modification of the aspect and modified aspect are fallen within the scope of the present invention.

### [Embodiment]

### (Treatment example 1)

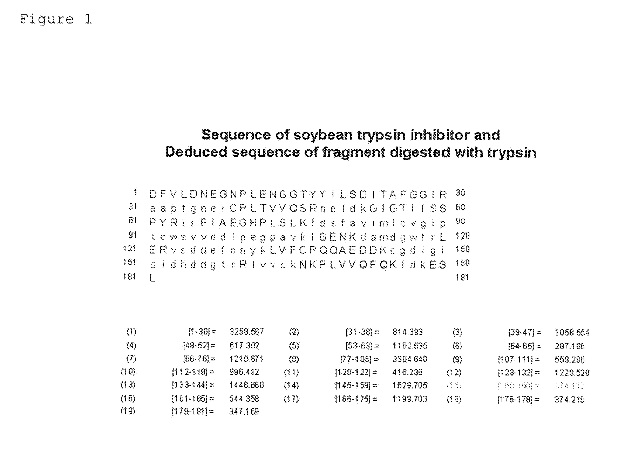

Freeze dried soybean trypsin inhibitor (sequence number 1) having an amino acid sequence as indicated in the upper portion of Figure 1 was mixed with methanol in the ratio of 10 mg:1 mL, and cooled at 5°C. 6 N of hydrochloric acid was dropwisely added to the mixture at 5°C under stirring so as to adjust the concentration as 0.01 N. After 16 hours, 6 N of hydrochloric acid was dropwisely and further added so as to adjust the concentration as 0.01 N. After another 24 hours, 6 N of hydrochloric acid was dropwisely and further added so as to adjust the concentration as 0.01 N, and stirred for another 3 days. Aliquote was batched off with pipetter, and dried under vacuum to remove the solvent, thereby obtaining Dry substance 1. It should be noted that lower portion of Figure 1 indicates number of residues from N terminal of the deduced peptide fragment of trypsin inhibitor digested with trypsin and the theoretical molecular weight thereof.

### (Reference example 1)

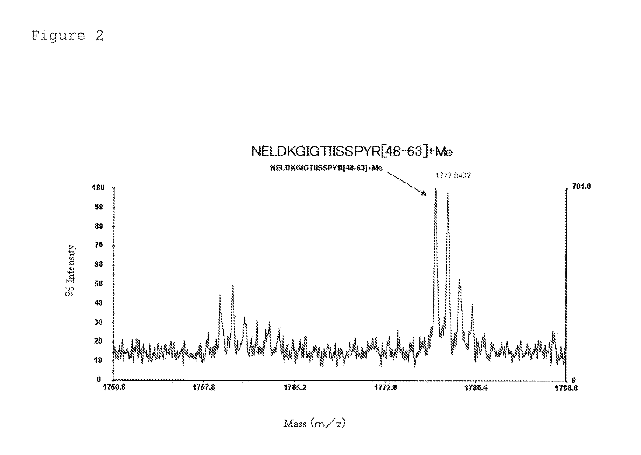

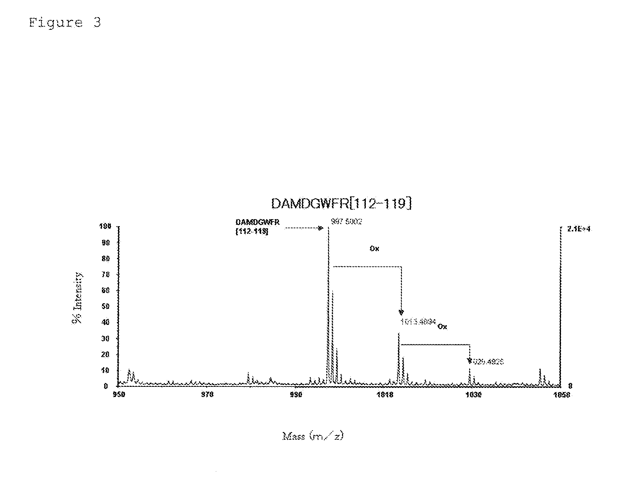

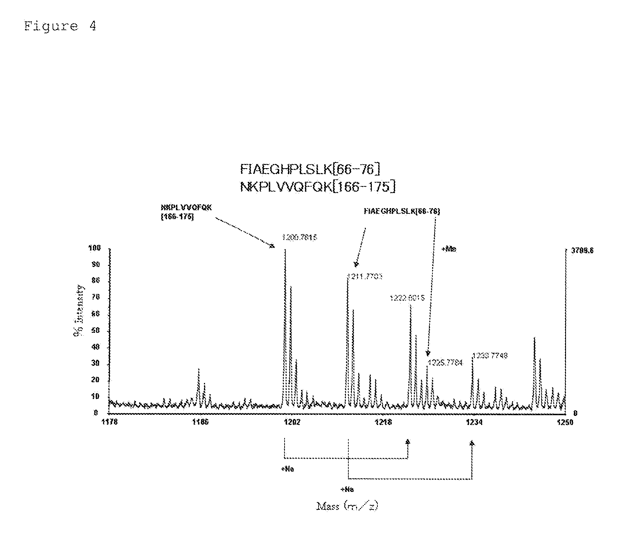

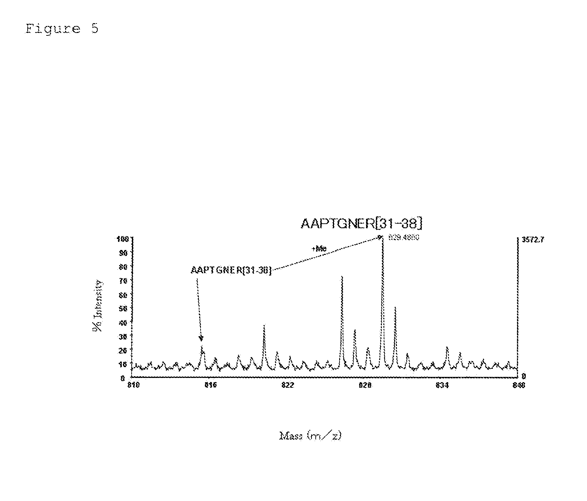

The Dry substance 1 as prepared in the Treatment example 1 was solved in buffer for polyacrylamide gel electrophoresis (SDS-PAGE) (50 mM of Tris-KCl (pH 6.8), 1% of 2-mercaptoethanol and 1% SDS) and SDS-PAGE was performed. The obtained band was cut into a size of 1 mm×1 mm, and immersed in 1.25 ng/µL of trypsin solution for 16 hours at 37°C to perform trypsin in-gel digestion. After that, each peptide fragment as eluted from the gel into the liquid phase was analyzed with MALDI-TOF-MS. The measurement was performed with positive ion mode and reflector mode. The obtained result was shown in Figures 2 to 5. Figures 2 to 5 show mass analysis spectrum of peptide fragment as obtained by digestion of trypsin inhibitor with trypsin, wherein Figure 2 corresponds to 48 to 63 residue (sequence number 21), Figure 3 corresponds to 112 to 119 residues (sequence number 22), Figure 4 corresponds to 66 to 76 residues (sequence number 24) and 167 to 175 residues (sequence number 23) and Figure 5 corresponds to 31 to 38 residues (sequence number 25) of peptide fragments, respectively. It should be noted that horizontal axis indicates mass number, and vertical axis indicates relative intensity (the same can be applied from Figure 2 to Figure 7).

A clearly in Figures 2 to 5, the peaks corresponding to the methyl derivatives was only observed in the peptide fragments having glutamic acid residue among each peptide fragment (sequence numbers 21, 23 and 25) as obtained by the trypsin digestion of trypsin inhibitor from the Treatment example 1 (in Figure 2, 1777.0432; in Figure 4, 1225.7784; and in Figure 5, 829.4880). Consequently, it was found that glutamic acid residues of these peptide fragments are methylated with the treatment of the Treatment example 1.

### (Reference example 2)

The peptide fragment corresponding to sequence number 26 which is obtained from a group of peptide fragments not subjected to the Treatment of the Treatment example 1 was separated with reverse phase column by nano LC, and then the obtained composition was analyzed with MS/MS. The result is shown in Figure 8. The difference between molecular weight of each peptide fragment containing glutamic acid residue at N terminal and theoretical molecular weight of peptide fragment as indicated in sequence number 26 (corresponding to b3 to b14 in Figure 8 and corresponding to sequence numbers 37 to 47, respectively) was 18. In addition, with regard to the molecular weight of the whole part, the peak less than 18 from the theoretical value was observed. Upon putting together with the result of Embodiment 1 as mentioned below, it is found that the glutamic acid residue at N terminal is dehydrated to form pyroglutamic acid residue.

### (Embodiment 1)

The Dry substance 1 as prepared in the Treatment example 1 was solved in the buffer for polyacrylamide gel electrophoresis (SDS-PAGE) (50 mM of Tris-KCl (pH 6.8), 1% of 2-mercaptoethanol and 1% SDS) and SDS-PAGE was performed. Then, the gel piece comprising trypsin inhibitor was cut into a size of 1 mm cubic, and a treatment of cleavage step as mentioned below was performed. The result is shown in Figure 6. Figure 6 (a) indicates mass analysis spectrum of the peptide fragment not subjected to the Treatment example 1, and Figure 6 (b) indicates mass analysis spectrum of the peptide fragment subjected to the Treatment example 1.

In Figure 6 (a), the peaks are found in mass numbers of 1673.69 and 1805.79, and correspond to hydrated derivative having the amino acid sequence as indicated in sequence number 26 and the amino acid sequence as indicated in sequence number 27, respectively. In addition, in Figure 6 (b), the peaks are found in mass numbers of 1673.97 and 1806.11, and correspond to the amino acid sequences of Figure 6 (a), respectively. As clearly from Figure 6, the peak intensity of the dehydrated derivative of the amino acid sequence as indicated in sequence number 26 was remarkably decreased in the group subjected to the treatment of the Treatment example 1. On the other hand, the peak intensity of the amino acid sequence as indicated in sequence number 27 was remarkably increased in the group subjected to the treatment of the Treatment example 1. Upon putting together with the result or Figure 2, it was found that the cleavage of glutamic acid residue at N terminal occurred in the treatment of the cleavage step is specifically suppressed by methylation of glutamic acid residue.

### (Embodiment 2)

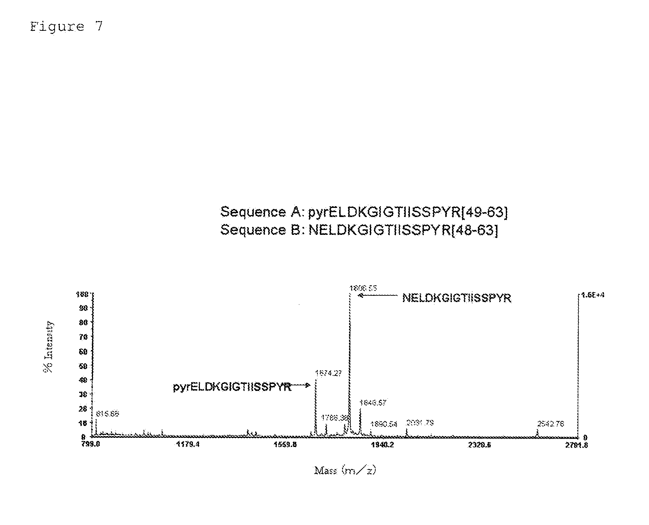

5 µg of trypsin inhibitor (sequence number 1) was electrophored with SDS-PAGE and the band was stained with CBB. The stained band was cut into a size of 1 mm×1 mm as gel species, and the gel species were immersed in a solution of water, methanol and TFA with a ratio of water: methanol: TFA=500:250:500(µL) and stood for 16 hours at room temperature. Then, the gel species were subjected to the treatment of cleavage step as mentioned below. The result is shown in Figure 7.

As clearly indicated in comparison with the result of Figure 6 (a), the cleavage reaction of glutamic acid residue at N terminal occurred in the cleavage step was remarkably suppressed by methylation of glutamic acid residue as obtained in the above-mentioned esterification step.

### <Treatment of cleavage step>

It was mobilized with 1.25 pmol/µL of glutaraldehyde solution. Next, the immobilized gel species were treated with pyridine solution, dehydrated with acetonitrile and immersed in 30% acetic acid anhydride in formamide solution for 1 hour at 60°C. Then, the gel species were immersed in 10% of dimethylamine in ethanol solution for 2 hours at 60°C to perform hydrolysis reaction. After that, in-gel digestion with trypsin digestion in accordance with the Reference example 1 was performed, and the obtained tryptic fragments were analyzed with MALDI-TOF-MS. The measurement was performed with positive ion mode and linear mode.

### (With regard to sequences as indicated in each sequence number)

The amino acid sequences as disclosed in the Specification, Claims and Drawing is listed as below in each sequence number.

The sequence number 1: The amino acid sequence of soybean trypsin inhibitor (Genbank No.1AVU, same as below)
The sequence number 2: The amino acid sequence of soybean trypsin inhibitor from 1 to 30 residues
The sequence number 3: The amino acid sequence of soybean trypsin inhibitor from 31 to 38 residues
The sequence number 4: The amino acid sequence of soybean trypsin inhibitor from 39 to 47 residues
The sequence number 5: The amino acid sequence of soybean trypsin inhibitor from 48 to 52 residues
The sequence number 6: The amino acid sequence of soybean trypsin inhibitor from 53 to 63 residues
The sequence number 7: The amino acid sequence of soybean trypsin inhibitor from 64 to 65 residues
The sequence number 8: The amino acid sequence of soybean trypsin inhibitor from 66 to 76 residues
The sequence number 9: The amino acid sequence of soybean trypsin inhibitor from 77 to 106 residues
The sequence number 10: The amino acid sequence of soybean trypsin inhibitor from 107 to 111 residues
The sequence number 11: The amino acid sequence of soybean trypsin inhibitor from 112 to 119 residues
The sequence number 12: The amino acid sequence of soybean trypsin inhibitor from 120 to 122 residues
The sequence number 13: The amino acid sequence of soybean trypsin inhibitor from 123 to 132 residues
The sequence number 14: The amino acid sequence of soybean trypsin inhibitor from 133 to 144 residues
The sequence number 15: The amino acid sequence of soybean trypsin inhibitor from 145 to 159 residues
The sequence number 16: The amino acid sequence of soybean trypsin inhibitor at 160 residue
The sequence number 17: The amino acid sequence of soybean trypsin inhibitor from 161 to 165 residues
The sequence number 18: The amino acid sequence of soybean trypsin inhibitor from 166 to 175 residues
The sequence number 19: The amino acid sequence of soybean trypsin inhibitor from 176 to 178 residues
The sequence number 20: The amino acid sequence of soybean trypsin inhibitor from 179 to 181 residues
The sequence number 21: The amino acid sequence of soybean trypsin inhibitor from 48 to 63 residues
The sequence number 22: The amino acid sequence of soybean trypsin inhibitor from 112 to 119 residues
The sequence number 23: The amino acid sequence of soybean trypsin inhibitor from 166 to 175 residues
The sequence number 24: The amino acid sequence of soybean trypsin inhibitor from 56 to 76 residues
The sequence number 25: The amino acid sequence of soybean trypsin inhibitor from 31 to 38 residues
The sequence number 26: The amino acid sequence of soybean trypsin inhibitor from 49 to 63 residues
The sequence number 27: The amino acid sequence of soybean trypsin inhibitor from 48 to 63 residues
The sequence number 28: The amino acid sequence of soybean trypsin inhibitor from 61 to 63 residues
The sequence number 29: The amino acid sequence of soybean trypsin inhibitor from 60 to 63 residues
The sequence number 30: The amino acid sequence of soybean trypsin inhibitor from 59 to 63 residues
The sequence number 31: The amino acid sequence of soybean trypsin inhibitor from 58 to 63 residues
The sequence number 32: The amino acid sequence of soybean trypsin inhibitor from 57 to 63 residues
The sequence number 33: The amino acid sequence of soybean trypsin inhibitor from 56 to 63 residues
The sequence number 34: The amino acid sequence of soybean trypsin inhibitor from 55 to 63 residues
The sequence number 35: The amino acid sequence of soybean trypsin inhibitor from 54 to 63 residues
The sequence number 36: The amino acid sequence of soybean trypsin inhibitor from 53 to 63 residues
The sequence number 37: The amino acid sequence of soybean trypsin inhibitor from 49 to 51 residues
The sequence number 38: The amino acid sequence of soybean trypsin inhibitor from 49 to 52 residues
The sequence number 39: The amino acid sequence of soybean trypsin inhibitor from 49 to 53 residues
The sequence number 40: The amino acid sequence of soybean trypsin inhibitor from 49 to 54 residues
The sequence number 41: The amino acid sequence of soybean trypsin inhibitor from 49 to 55 residues
The sequence number 42: The amino acid sequence of soybean trypsin inhibitor from 49 to 56 residues
The sequence number 43: The amino acid sequence of soybean trypsin inhibitor from 49 to 57 residues
The sequence number 44: The amino acid sequence of soybean trypsin inhibitor from 49 to 58 residues
The sequence number 45: The amino acid sequence of soybean trypsin inhibitor from 49 to 59 residues
The sequence number 46: The amino acid sequence of soybean trypsin inhibitor from 49 to 60 residues
The sequence number 47: The amino acid sequence of soybean trypsin inhibitor from 49 to 62 residues

The present invention was explained with the preferred embodiment of the present invention. It is obvious that various modification and changes to these embodiments can be made without departing from the spirit and scope of the present invention as defined in the Claims, although the present invention was explained with reference to the particular embodiment. That is, it should not be construed that the present invention is limited to the detailed particular embodiment and the attached Drawing.

## Claims

1. A method for cleavage of peptidic bond at C terminal of peptide comprising the steps of:
reacting a peptide with a solution of perhalogenated carboxylic acid containing alcohol to esterify glutamic acid residue of said peptide; and
reacting said peptide with alkanoic acid anhydride to obtain C terminal-deleted peptide in which the amino acid residue of said peptide at C terminal is sequentially deleted.

2. The method according to Claim 1, wherein said step of reacting the peptide with the solution of perhalogenated carboxylic acid containing alcohol is a step of performing said peptide supported in a substrate.

3. The method according to Claim 2 wherein the substrate is polyacrylamide gel.

4. The method according to any one of Claims 1 to 3, wherein the solution of perhalogenated carboxylic acid containing alcohol is a solution selected from the group consisting of an aqueous solution and an alcohol solution.

5. The method according to any one of Claims 1 to 4, wherein said alcohol has 1 to 5 carbon atoms.

6. The method according to Claim 5, wherein said alcohol is methanol.

7. The method according to any one of Claims 1 to 6, wherein said perhalogenated carboxylic acid is selected from the group consisting of trichloroacetic acid, trifluoroacetic acid, pentafluoropropionic acid and heptafluorobutyric acid.

8. The method according to Claim 7, wherein said perhalogenated carboxylic acid is trifluoroacetic acid.

9. The method according to any one of Claims 1 to 8, wherein the solution of said perhalogenated carboxylic acid has a ratio of 100 to 600: 150 to 350: 300 to 700 in volume of water: methanol: TFA.

10. A method for determination of C terminal amino acid sequence of peptide comprising the steps of:
reacting a peptide with a solution of perhalogenated carboxylic acid containing alcohol to esterify glutamic acid residue of said peptide;
reacting said peptide with alkanoic acid anhydride to obtain C terminal-deleted peptide in which the amino acid residue of said peptide at C terminal is sequentially deleted;
measuring molecular weight of said C terminal-deleted peptide; and
grasping said peptide based on said molecular weight.

11. The method according to Claim 10, wherein said step of grasping said peptide based on said molecular weight is a step of grasping C terminal amino acid sequence of said peptide based on the difference between the molecular weight of said peptide and molecular weight of said C terminal-deleted peptide.

12. A method for determination of C terminal amino acid sequence of peptide comprising the steps of:
reacting a peptide with a solution of perhalogenated carboxylic acid containing alcohol to esterify glutamic acid residue of said peptide;
reacting said peptide with alkanoic acid anhydride to obtain C terminal-deleted peptide in which the amino acid residue of said peptide at C terminal is sequentially deleted;
reacting said peptide with site specific cleaving agent to obtain a C terminal-deleted peptide originated peptide fragment originated from said C terminal-deleted peptide;
measuring molecular weight of said C terminal-deleted peptide originated peptide fragment; and
grasping said peptide based on said molecular weight.

13. The method according to Claim 12, wherein said site specific cleaving agent is a agent selected from the group consisting of trypsin, V8 proteasse and CNBr.

14. A method for determination of C terminal amino acid sequence of peptide supported in a substrate comprising the steps of:
reacting a peptide supported in a substrate with a solution of perhalogenated carboxylic acid containing alcohol to esterify glutamic acid residue of said peptide;
reacting said peptide with alkanoic acid anhydride to obtain C terminal-deleted peptide in which the amino acid residue of said peptide at C terminal is sequentially deleted;
immersing said substrate into a solution containing site specific cleaving agent to obtain a C terminal-deleted peptide originated peptide fragment originated from said C terminal-deleted peptide;
measuring molecular weight of said C terminal-deleted peptide originated peptide fragment; and
grasping said peptide based on said molecular weight.

15. The method according to Claim 14, wherein said step of grasping said peptide based on said molecular weight is a step of grasping C terminal amino acid sequence of said peptide based on the difference between molecular weight of said C terminal-deleted peptide originated peptide fragment, and molecular weight of peptide fragment corresponding to said peptide fragment and originated from said peptide.

16. The method according to Claim 14 or 15, wherein the solution of said perhalogenated carboxylic acid containing alcohol has a ratio of 150 to 350: 300 to 700 in volume of methanol: TFA.

17. The method according to any one of Claims 14 to 16, wherein said site specific cleaving agent is a agent elected from the group consisting of trypsin, V8 proteasse and CNBr.

18. The method according to any one of Claims 14 to 17, wherein the substrate is polyacrylamide gel.


SEQUENCE LISTING

<110>  NEC Corporation

<120>  A method for cleavage of peptidic bond at C terminal of
peptide and method for determination of C terminal amino acid
sequence of peptide

<130>  341-04024

<150>  JP2006-031097
<151>  2006-02-08

<160>  47

<170>  PatentIn version 3.1

<210>  1
<211>  181
<212>  PRT
<213>  Glycine max

<400>  1

Asp Phe Val Leu Asp Asn Glu Gly Asn Pro Leu Glu Asn Gly Gly Thr
1               5                   10                  15

Tyr Tyr Ile Leu Ser Asp Ile Thr Ala Phe Gly Gly Ile Arg Ala Ala
            20                  25                  30

Pro Thr Gly Asn Glu Arg Cys Pro Leu Thr Val Val Gln Ser Arg Asn
        35                  40                  45

Glu Leu Asp Lys Gly Ile Gly Thr Ile Ile Ser Ser Pro Tyr Arg Ile
    50                  55                  60

Arg Phe Ile Ala Glu Gly His Pro Leu Ser Leu Lys Phe Asp Ser Phe
65                  70                  75                  80

Ala Val Ile Met Leu Cys Val Gly Ile Pro Thr Glu Trp Ser Val Val
                85                  90                  95

Glu Asp Leu Pro Glu Gly Pro Ala Val Lys Ile Gly Glu Asn Lys Asp
            100                 105                 110

Ala Met Asp Gly Trp Phe Arg Leu Glu Arg Val Ser Asp Asp Glu Phe
        115                 120                 125

Asn Asn Tyr Lys Leu Val Phe Cys Pro Gln Gln Ala Glu Asp Asp Lys
    130                 135                 140

Cys Gly Asp Ile Gly Ile Ser Ile Asp His Asp Asp Gly Thr Arg Arg
145                 150                 155                 160

Leu Val Val Ser Lys Asn Lys Pro Leu Val Val Gln Phe Gln Lys Leu
                165                 170                 175

Asp Lys Glu Ser Leu
            180

<210> 2
<211> 30
<212> PRT
<213> Glycine max

<400> 2

Asp Phe Val Leu Asp Asn Glu Gly Asn Pro Leu Glu Asn Gly Gly Thr
1               5                   10                  15

Tyr Tyr Ile Leu Ser Asp Ile Thr Ala Phe Gly Gly Ile Arg
            20                  25                  30

<210> 3
<211> 8
<212> PRT
<213> Glycine max

<400> 3

Ala Ala Pro Thr Gly Asn Glu Arg
1               5

<210> 4
<211> 9
<212> PRT
<213> Glycine max

<400> 4

Cys Pro Leu Thr Val Val Gln Ser Arg
1               5

<210>  5
<211>  4
<212>  PRT
<213>  Glycine max

<400>  5

Glu Leu Asp Lys
1

<210>  6
<211>  11
<212>  PRT
<213>  Glycine max

<400>  6

Gly Ile Gly Thr Ile Ile Ser Ser Pro Tyr Arg
1               5                   10

<210>  7
<211>  2
<212>  PRT
<213>  Glycine max

<400>  7

Ile Arg
1

<210>  8
<211>  11

<212>  PRT
<213>  Glycine max

<400>  8

Phe Ile Ala Glu Gly His Pro Leu Ser Leu Lys
1               5                   10

<210>  9
<211>  30
<212>  PRT
<213>  Glycine max

<400>  9

Phe Asp Ser Phe Ala Val Ile Met Leu Cys Val Gly Ile Pro Thr Glu
1               5                   10                  15

Trp Ser Val Val Glu Asp Leu Pro Glu Gly Pro Ala Val Lys
            20                  25                  30

<210>  10
<211>  5
<212>  PRT
<213>  Glycine max

<400>  10

Ile Gly Glu Asn Lys
1               5

<210>  11

<211>  8
<212>  PRT
<213>  Glycine max

<400>  11

Asp Ala Met Asp Gly Trp Phe Arg
1               5

<210>  12
<211>  3
<212>  PRT
<213>  Glycine max

<400>  12

Leu Glu Arg
1

<210>  13
<211>  10
<212>  PRT
<213>  Glycine max

<400>  13

Val Ser Asp Asp Glu Phe Asn Asn Tyr Lys
1               5                   10

<210>  14
<211>  12
<212>  PRT
<213>  Glycine max

<400> 14

Leu Val Phe Cys Pro Gln Gln Ala Glu Asp Asp Lys
1               5                   10

<210> 15
<211> 15
<212> PRT
<213> Glycine max

<400> 15

Cys Gly Asp Ile Gly Ile Ser Ile Asp His Asp Asp Gly Thr Arg
1               5                   10                  15

<210> 16
<211> 1
<212> PRT
<213> Glycine max

<400> 16

Arg
1

<210> 17
<211> 5
<212> PRT
<213> Glycine max

<400> 17

Leu Val Val Ser Lys
1               5

<210>  18
<211>  10
<212>  PRT
<213>  Glycine max

<400>  18

Asn Lys Pro Leu Val Val Gln Phe Gln Lys
1               5                   10

<210>  19
<211>  3
<212>  PRT
<213>  Glycine max

<400>  19

Leu Asp Lys
1

<210>  20
<211>  3
<212>  PRT
<213>  Glycine max

<400>  20

Glu Ser Leu
1

<210> 21

<211> 16

<212> PRT

<213> Glycine max

<400> 21

Asn Glu Leu Asp Lys Gly Ile Gly Thr Ile Ile Ser Ser Pro Tyr Arg
1               5                   10                  15

<210> 22

<211> 8

<212> PRT

<213> Glycine max

<400> 22

Asp Ala Met Asp Gly Trp Phe Arg
1               5

<210> 23

<211> 10

<212> PRT

<213> Glycine max

<400> 23

Asn Lys Pro Leu Val Val Gln Phe Gln Lys
1               5                   10

<210> 24

<211> 11

<212>  PRT
<213>  Glycine max

<400>  24

Phe Ile Ala Glu Gly His Pro Leu Ser Leu Lys
1               5                   10

<210>  25
<211>  8
<212>  PRT
<213>  Glycine max

<400>  25

Ala Ala Pro Thr Gly Asn Glu Arg
1               5

<210>  26
<211>  15
<212>  PRT
<213>  Glycine max

<400>  26

Glu Leu Asp Lys Gly Ile Gly Thr Ile Ile Ser Ser Pro Tyr Arg
1               5                   10                  15

<210>  27
<211>  16
<212>  PRT
<213>  Glycine max

<400> 27

Asn Glu Leu Asp Lys Gly Ile Gly Thr Ile Ile Ser Ser Pro Tyr Arg
1               5                   10                  15

<210> 28
<211> 3
<212> PRT
<213> Glycine max

<400> 28

Pro Tyr Arg
1

<210> 29
<211> 4
<212> PRT
<213> Glycine max

<400> 29

Ser Pro Tyr Arg
1

<210> 30
<211> 5
<212> PRT
<213> Glycine max

<400> 30

Ser Ser Pro Tyr Arg

1               5

<210>  31
<211>  6
<212>  PRT
<213>  Glycine max

<400>  31

Ile Ser Ser Pro Tyr Arg
1               5

<210>  32
<211>  7
<212>  PRT
<213>  Glycine max

<400>  32

Ile Ile Ser Ser Pro Tyr Arg
1               5

<210>  33
<211>  8
<212>  PRT
<213>  Glycine max

<400>  33

Thr Ile Ile Ser Ser Pro Tyr Arg
1               5

<210> 34

<211> 9

<212> PRT

<213> Glycine max

<400> 34

Gly Thr Ile Ile Ser Ser Pro Tyr Arg
1               5

<210> 35

<211> 10

<212> PRT

<213> Glycine max

<400> 35

Ile Gly Thr Ile Ile Ser Ser Pro Tyr Arg
1               5                   10

<210> 36

<211> 11

<212> PRT

<213> Glycine max

<400> 36

Gly Ile Gly Thr Ile Ile Ser Ser Pro Tyr Arg
1               5                   10

<210> 37

<211> 3

<212> PRT

<213>  Glycine max

<400>  37

Glu Leu Asp
1

<210>  38
<211>  4
<212>  PRT
<213>  Glycine max

<400>  38

Glu Leu Asp Lys
1

<210>  39
<211>  5
<212>  PRT
<213>  Glycine max

<400>  39

Glu Leu Asp Lys Gly
1               5

<210>  40
<211>  6
<212>  PRT
<213>  Glycine max

<400>  40

Glu Leu Asp Lys Gly Ile
1               5

<210>  41
<211>  7
<212>  PRT
<213>  Glycine max

<400>  41

Glu Leu Asp Lys Gly Ile Gly
1               5

<210>  42
<211>  8
<212>  PRT
<213>  Glycine max

<400>  42

Glu Leu Asp Lys Gly Ile Gly Thr
1               5

<210>  43
<211>  9
<212>  PRT
<213>  Glycine max

<400>  43

Glu Leu Asp Lys Gly Ile Gly Thr Ile
1               5

<210> 44
<211> 10
<212> PRT
<213> Glycine max

<400> 44

Glu Leu Asp Lys Gly Ile Gly Thr Ile Ile
1               5                   10

<210> 45
<211> 11
<212> PRT
<213> Glycine max

<400> 45

Glu Leu Asp Lys Gly Ile Gly Thr Ile Ile Ser
1               5                   10

<210> 46
<211> 12
<212> PRT
<213> Glycine max

<400> 46

Glu Leu Asp Lys Gly Ile Gly Thr Ile Ile Ser Ser
1               5                   10

<210> 47

<211>  14

<212>  PRT

<213>  Glycine max

<400>  47

Glu Leu Asp Lys Gly Ile Gly Thr Ile Ile Ser Ser Pro Tyr
1               5                   10

SEQUENCE LISTING

<110> NEC Corporation

<120> A method for cleavage of peptidic bond at C terminal of
peptide and method for determination of C terminal amino acid
sequence of peptide

<130> 341-04024

<150> JP2006-031097
<151> 2006-02-08

<160> 47

<170> PatentIn version 3.1

<210> 1
<211> 181
<212> PRT
<213> Glycine max

<400> 1

Asp Phe Val Leu Asp Asn Glu Gly Asn Pro Leu Glu Asn Gly Gly Thr
1               5                   10                  15

Tyr Tyr Ile Leu Ser Asp Ile Thr Ala Phe Gly Gly Ile Arg Ala Ala
            20                  25                  30

Pro Thr Gly Asn Glu Arg Cys Pro Leu Thr Val Val Gln Ser Arg Asn
        35                  40                  45

Glu Leu Asp Lys Gly Ile Gly Thr Ile Ile Ser Ser Pro Tyr Arg Ile
    50                  55                  60

Arg Phe Ile Ala Glu Gly His Pro Leu Ser Leu Lys Phe Asp Ser Phe
65                  70                  75                  80

Ala Val Ile Met Leu Cys Val Gly Ile Pro Thr Glu Trp Ser Val Val
                85                  90                  95

Glu Asp Leu Pro Glu Gly Pro Ala Val Lys Ile Gly Glu Asn Lys Asp
            100                 105                 110

Ala Met Asp Gly Trp Phe Arg Leu Glu Arg Val Ser Asp Asp Glu Phe
        115                 120                 125

Asn Asn Tyr Lys Leu Val Phe Cys Pro Gln Gln Ala Glu Asp Asp Lys
    130                 135                 140

Cys Gly Asp Ile Gly Ile Ser Ile Asp His Asp Asp Gly Thr Arg Arg
145                 150                 155                 160

Leu Val Val Ser Lys Asn Lys Pro Leu Val Val Gln Phe Gln Lys Leu
                165                 170                 175

Asp Lys Glu Ser Leu
            180

<210> 2
<211> 30
<212> PRT
<213> Glycine max

<400> 2

Asp Phe Val Leu Asp Asn Glu Gly Asn Pro Leu Glu Asn Gly Gly Thr
1               5                   10                  15

Tyr Tyr Ile Leu Ser Asp Ile Thr Ala Phe Gly Gly Ile Arg
            20                  25                  30

<210> 3
<211> 8
<212> PRT
<213> Glycine max

<400> 3

Ala Ala Pro Thr Gly Asn Glu Arg
1               5

<210> 4
<211> 9
<212> PRT
<213> Glycine max

<400> 4

Cys Pro Leu Thr Val Val Gln Ser Arg
1               5

<210> 5

<211> 4

<212> PRT

<213> Glycine max

<400> 5

Glu Leu Asp Lys
1

<210> 6

<211> 11

<212> PRT

<213> Glycine max

<400> 6

Gly Ile Gly Thr Ile Ile Ser Ser Pro Tyr Arg
1               5                   10

<210> 7

<211> 2

<212> PRT

<213> Glycine max

<400> 7

Ile Arg
1

<210> 8

<211> 11

<212>  PRT
<213>  Glycine max

<400>  8

Phe Ile Ala Glu Gly His Pro Leu Ser Leu Lys
1               5                   10

<210>  9
<211>  30
<212>  PRT
<213>  Glycine max

<400>  9

Phe Asp Ser Phe Ala Val Ile Met Leu Cys Val Gly Ile Pro Thr Glu
1               5                   10                  15

Trp Ser Val Val Glu Asp Leu Pro Glu Gly Pro Ala Val Lys
            20                  25                  30

<210>  10
<211>  5
<212>  PRT
<213>  Glycine max

<400>  10

Ile Gly Glu Asn Lys
1               5

<210>  11

<211>  8
<212>  PRT
<213>  Glycine max

<400>  11

Asp Ala Met Asp Gly Trp Phe Arg
1               5

<210>  12
<211>  3
<212>  PRT
<213>  Glycine max

<400>  12

Leu Glu Arg
1

<210>  13
<211>  10
<212>  PRT
<213>  Glycine max

<400>  13

Val Ser Asp Asp Glu Phe Asn Asn Tyr Lys
1               5                   10

<210>  14
<211>  12
<212>  PRT
<213>  Glycine max

<400> 14

Leu Val Phe Cys Pro Gln Gln Ala Glu Asp Asp Lys
1               5                   10

<210> 15
<211> 15
<212> PRT
<213> Glycine max

<400> 15

Cys Gly Asp Ile Gly Ile Ser Ile Asp His Asp Asp Gly Thr Arg
1               5                   10                  15

<210> 16
<211> 1
<212> PRT
<213> Glycine max

<400> 16

Arg
1

<210> 17
<211> 5
<212> PRT
<213> Glycine max

<400> 17

Leu Val Val Ser Lys
1               5

<210>  18
<211>  10
<212>  PRT
<213>  Glycine max

<400>  18

Asn Lys Pro Leu Val Val Gln Phe Gln Lys
1               5                   10

<210>  19
<211>  3
<212>  PRT
<213>  Glycine max

<400>  19

Leu Asp Lys
1

<210>  20
<211>  3
<212>  PRT
<213>  Glycine max

<400>  20

Glu Ser Leu
1

<210> 21
<211> 16
<212> PRT
<213> Glycine max

<400> 21

Asn Glu Leu Asp Lys Gly Ile Gly Thr Ile Ile Ser Ser Pro Tyr Arg
1               5                   10                  15

<210> 22
<211> 8
<212> PRT
<213> Glycine max

<400> 22

Asp Ala Met Asp Gly Trp Phe Arg
1               5

<210> 23
<211> 10
<212> PRT
<213> Glycine max

<400> 23

Asn Lys Pro Leu Val Val Gln Phe Gln Lys
1               5                   10

<210> 24
<211> 11

<212>  PRT
<213>  Glycine max

<400>  24

Phe Ile Ala Glu Gly His Pro Leu Ser Leu Lys
1               5                   10

<210>  25
<211>  8
<212>  PRT
<213>  Glycine max

<400>  25

Ala Ala Pro Thr Gly Asn Glu Arg
1               5

<210>  26
<211>  15
<212>  PRT
<213>  Glycine max

<400>  26

Glu Leu Asp Lys Gly Ile Gly Thr Ile Ile Ser Ser Pro Tyr Arg
1               5                   10                  15

<210>  27
<211>  16
<212>  PRT
<213>  Glycine max

<400> 27

Asn Glu Leu Asp Lys Gly Ile Gly Thr Ile Ile Ser Ser Pro Tyr Arg
1               5                   10                  15

<210> 28
<211> 3
<212> PRT
<213> Glycine max

<400> 28

Pro Tyr Arg
1

<210> 29
<211> 4
<212> PRT
<213> Glycine max

<400> 29

Ser Pro Tyr Arg
1

<210> 30
<211> 5
<212> PRT
<213> Glycine max

<400> 30

Ser Ser Pro Tyr Arg

1               5

<210>  31
<211>  6
<212>  PRT
<213>  Glycine max

<400>  31

Ile Ser Ser Pro Tyr Arg
1               5

<210>  32
<211>  7
<212>  PRT
<213>  Glycine max

<400>  32

Ile Ile Ser Ser Pro Tyr Arg
1               5

<210>  33
<211>  8
<212>  PRT
<213>  Glycine max

<400>  33

Thr Ile Ile Ser Ser Pro Tyr Arg
1               5

<210> 34
<211> 9
<212> PRT
<213> Glycine max

<400> 34

Gly Thr Ile Ile Ser Ser Pro Tyr Arg
1               5

<210> 35
<211> 10
<212> PRT
<213> Glycine max

<400> 35

Ile Gly Thr Ile Ile Ser Ser Pro Tyr Arg
1               5                   10

<210> 36
<211> 11
<212> PRT
<213> Glycine max

<400> 36

Gly Ile Gly Thr Ile Ile Ser Ser Pro Tyr Arg
1               5                   10

<210> 37
<211> 3
<212> PRT

<213>  Glycine max

<400>  37

Glu Leu Asp
1

<210>  38
<211>  4
<212>  PRT
<213>  Glycine max

<400>  38

Glu Leu Asp Lys
1

<210>  39
<211>  5
<212>  PRT
<213>  Glycine max

<400>  39

Glu Leu Asp Lys Gly
1               5

<210>  40
<211>  6
<212>  PRT
<213>  Glycine max

<400>  40

Glu Leu Asp Lys Gly Ile
1               5

<210>  41
<211>  7
<212>  PRT
<213>  Glycine max

<400>  41

Glu Leu Asp Lys Gly Ile Gly
1               5

<210>  42
<211>  8
<212>  PRT
<213>  Glycine max

<400>  42

Glu Leu Asp Lys Gly Ile Gly Thr
1               5

<210>  43
<211>  9
<212>  PRT
<213>  Glycine max

<400>  43

Glu Leu Asp Lys Gly Ile Gly Thr Ile
1               5

<210>  44
<211>  10
<212>  PRT
<213>  Glycine max

<400>  44

Glu Leu Asp Lys Gly Ile Gly Thr Ile Ile
1               5                   10

<210>  45
<211>  11
<212>  PRT
<213>  Glycine max

<400>  45

Glu Leu Asp Lys Gly Ile Gly Thr Ile Ile Ser
1               5                   10

<210>  46
<211>  12
<212>  PRT
<213>  Glycine max

<400>  46

Glu Leu Asp Lys Gly Ile Gly Thr Ile Ile Ser Ser
1               5                   10

<210>  47

<211>  14

<212>  PRT

<213>  Glycine max

<400>  47

Glu Leu Asp Lys Gly Ile Gly Thr Ile Ile Ser Ser Pro Tyr
1               5                   10